# FASCICULE DE BREVET EUROPEEN

(11) **EP 2 014 689 B1**
(45) Date de publication et mention de la délivrance du brevet: **05.10.2011**
(21) Numéro de dépôt: 08166125.8
(22) Date de dépôt: 26.01.2004
(51) Int. Cl.: C08F 220/60, C08F 246/00, A61K 8/81, A61Q 5/12, C08F 2/32, C08F 220/56, C08L 33/26, C08F 220/28

(54) **Nouveaux épaississants cationiques, procédé pour leur préparation et composition en contenant**
Neue kationische Verdickungsmittel, ihr Herstellungsverfahren und Zusammensetzung, die sie enthält
New cationic thickeners, method for preparing them and composition containing them

(30) Priorité: 13.02.2003 FR 0301723
(43) Date de publication de la demande: 14.01.2009
(62) Demande divisionnaire de: 04300043.9
(73) Titulaire: Societe D'Exploitation De Produits Pour Les Industries Chimiques Seppic, 75007 Paris (FR)
(72) Inventeur: Braun, Olivier, 81100 CASTRES (FR); Mallo, Paul, 78290 CROISSY- SUR -SEINE (FR); Rolland, Hervé, 81100 CASTRES (FR)
(74) Mandataire: Conan, Philippe Claude

(56) Documents cités:
- EP-A1- 0 915 107
- EP-A1- 1 323 753
- WO-A-97/22640
- FR-A1- 2 798 851
- US-A- 4 981 936

## Description

L'invention a pour objet, de nouveaux polymères leur procédé de préparation ainsi que leur utilisation comme agent épaississant et/ou émulsionnant.

L'épaississement des phases aqueuses est en général réalisé en y incorporant des polymères hydrophiles de toutes sortes, qu'ils soient synthétiques ou d'origine naturelle. Parmi les polymères d'origine naturelle, les gommes de xanthane ou de guar sont assez largement utilisées. Ils ont cependant les inconvénients classiques des produits naturels, à savoir une qualité et un prix fluctuant.

Parmi les épaississants synthétiques hydrophiles les plus largement utilisés, il y a les polymères sous forme de poudres ou de latex inverses auto-inversibles. Ils sont mis en oeuvre dans une large gamme de pH et sont souvent bien tolérés par l'homme. De telles compositions sont décrites par exemple dans les demandes de brevet et brevets français publiés sous les numéros 2721511, 2773805, 2774688, 2774996, 2782086, 2785801, 2786493, 2787457, 2789395, 2794034, 2794124, 2808446, 2808447 et 2810883.

Ces polymères sont anioniques et sont essentiellement destinés à épaissir et/ou à émulsionner des formulations topiques cosmétiques, dermopharmaceutiques ou pharmaceutiques, qui contiennent de nombreux constituants comme des huiles, des tensioactifs, non ioniques ou anioniques, des sels minéraux et/ou des acides faibles.

Certaines formulations, notamment celles destinées au soin capillaire, contiennent aussi des tensioactifs cationiques et ou des polymères conditionneurs cationiques. Dans ce cas particulier, les épaississants constitués par des polymères anioniques ne sont pas recommandés à cause des interactions électrostatiques entre les charges positives et négatives qui provoquent une précipitation du polymère et on utilise de préférence des polymères épaississants cationiques comme ceux décrits dans les brevets américains publiés sous les numéros US 4 806 345 et US 5 100 660.

Bien que ces derniers se comportent de manière satisfaisante en milieu acide et qu'ils soient compatibles avec les tensioactifs cationiques, ils perdent néanmoins de leur pouvoir épaississant dans des formulations riches en électrolytes.

Le brevet américain US 4 981 936 divulgue des terpolymères d'acrylate polyéthoxylés, d'acrylamide et de monomères cationiques.

La demande internationale publiée sous le numéro WO97/22640 divulgue des dispersions aqueuses de polymères tensioactifs comprenant un monomères cationique vinylique, un monomère vinylique ayant une fonction amide et un monomère vinylique possédant à la fois un groupe hydrophile et un groupe hydrophobe.

C'est pourquoi .la demanderesse s'est attachée à développer de nouveaux épaississants de nature cationique, qui soient compatibles avec les tensioactifs cationiques tout en conservant leur pourvoir épaississant dans les milieux riches en électrolytes.

Selon un premier aspect, l'invention a pour objet un polyélectrolyte cationique linéaire ou réticulé, caractérisé en ce qu'il est obtenu par copolymérisation d'au moins un monomère cationique avec au moins un monomère neutre et au moins un monomère tensioactif non ionique, choisi parmi les composés représentés par la formule générale (I) :

A-C(=O)-O-[(CH₂-CH(R₁)-O]ₙ-R (I)

dans laquelle :
- A représente le radical vinyle ou le radical 2-propènyle ;
- R représente un radical aliphatique hydrocarboné, linéaire ou ramifié, saturé ou insaturé comprenant de 8 à 18 atomes de carbone ;
- R₁ représente un atome d'hydrogène ;
- n représente un nombre compris entre 1 et 10 ;
et dans lequel :
- de 5 % à 35 % des motifs monomériques qu'il comprend, est un monomère cationique,
- de 55 % à 95 % des motifs monomériques qu'il comprend, est un monomère neutre, et
- de 0,1 % à 5 % des motifs monomériques qu'il comprend, est un monomère tensioactif non ionique de formule (I).

Par polyélectrolyte réticulé, on désigne un polyélectrolyte non linéaire se présentant à l'état de réseau tridimensionnel insoluble dans l'eau, mais gonflable dans l'eau et conduisant donc à l'obtention d'un gel chimique.

Par copolymérisation, on signifie dans le cadre de la présente invention, que la réaction de polymérisation met en oeuvre au moins trois monomères différents. Elle peut cependant impliquer plus de trois monomères différents.

Par monomère neutre, on désigne des monomères ne comportant aucune fonction acide fort ou faible ni aucun groupe chargé positivement. Ils sont plus particulièrement choisis parmi l'acrylamide, le méthacrylamide, le vinylpyrrolidone, le diacétoneacrylamide, le diméthylacrylamide, l'acrylate de (2-hydroxy éthyle), l'acrylate de (2,3dihydroxy propyle), le méthacrylate de (2-hydroxy éthyle), le méthacrylate de (2,3dihydroxy propyle) ou un dérivé éthoxylé de poids moléculaire compris entre 400 et 1000, de chacun de ces esters.

Par monomères cationiques, on désigne plus particulièrement des monomères comportant une fonction ammonium quaternaire. Ils sont plus particulièrement choisis parmi le chlorure de 2,N,N,N-tétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,N-triméthyl 3-[(1-oxo 2-propènyl) amino] propanammonium (APTAC), le chlorure de diallyl diméthylammonium (DADMAC), le chlorure de N,N,N-triméthyl 2-[(1-oxo 2-propènyl)] éthanammonium, le chlorure de N,N,N-triméthyl 2-[(1-oxo 2- méthyl 2-propènyl)] éthanammonium, le N-[2-(diméthylamillo) 1,1-diméthyl] acrylamide, le N-[2-(méthylamino) 1,1-diméthyl] acrylamide, l'acrylate de 2-(diméthylamino) éthyle, le méthacrylate de 2-(diméthylamino) éthyle ou le N-[3-(diméthylamino) propyl] acrylamide.

Par radical aliphatique, hydrocarboné linéaire, saturé ou insaturé, comprenant de 8 à 18 atomes de carbone, on désigne plus particulièrement pour R, les radicaux dérivés des alcools primaires linéaires tels que par exemple, ceux dérivés des alcools octylique, pélargonique, décylique, undécylique, undécénylique, laurique, tridécylique, myristylique, pentadécylique, cétylique, heptadécylique, stéarylique, oléylique, linoléylique,. Il s'agit alors des radicaux octyle, nonyle, décyle, undécyle, 10-undécènyle, dodécyle, tridécyle, tétradécyle, pentadécyle, hexadécyle, heptadécyle, octadécyle, 9-octadécènyle ou 10,12-octadécadiènyle,.

Par radical aliphatique hydrocarboné ramifié, saturé ou insaturé comprenant de 8 à 18 atomes de carbone, on désigne plus particulièrement pour R :
soit les radicaux dérivés des alcools de Guerbet, qui sont des 1-alcanols ramifiés répondant à la formule générale :

   CH₃-(CH₂)ₚ-CH[CH₃-(CH₂)ₚ₋₂]-CH₂OH,

   dans laquelle p représente un nombre entier compris entre 2 et 8, tels que, par exemple, les radicaux 2-éthyl hexyle, 2-propyl heptyle, 2-butyl octyle, 2-pentyl nonyle ou 2-hexyl décyle ;
soit les radicaux dérivés des isoalcanols répondant à la formule générale :

   CH₃-CH(CH₃)-(CH₂)ₘ-CH₂OH,
dans laquelle m représente un nombre entier compris entre 2 et 14 tels que, par exemple, les radicaux 4-méthyl pentyle, 5-méthyl hexyle, 6-méthyl heptyle, 15-méthyl pendadécyle ou 16-méthyl heptadécyle ;
soit les radicaux 2-hexyl octyle, 2-octyl décyle ou 2-hexyl dodécyle.

Selon un autre aspect particulier de la présente invention, le polyélectrolyte tel que défini précédemment est réticulé. Dans ce dernier cas l'agent de réticulation est choisi notamment parmi les composés diéthyléniques ou polyéthyléniques, et tout particulièrement parmi l'acide diallyloxyacétique ou un des sels et notamment son sel de sodium, le triallylamine, le triméthylol propanetriacrylate, le diméthacrylate d'éthylèneglycol, le diacrylate de diéthylène glycol, le diallylurée ou le méthylène bis(acrylamide).

L'agent de réticulation est alors généralement mis en oeuvre dans la proportion molaire exprimée par rapport aux monomères mis en oeuvre, de 0,005 % à 1 %, notamment de 0,01 % à 0,2 % et plus particulièrement de 0,01 % à 0,1 %.

Selon un deuxième aspect de la présente invention, celle-ci a pour objet une composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20 % à 70 % en poids, et de préférence de 25 % à 40 % en poids, d'un polyélectrolyte cationique tel que défini précédemment.

Le latex inverse auto-inversible selon l'invention contient généralement de 2,5 % à 15 % en poids, et de préférence de 4 % à 9 % en poids, d'agents émulsifiants, parmi lesquels de 20 % à 50 %, notamment de 25 % à 40 % du poids total des agents émulsifiants présents sont du type eau dans huile (E/H) et dans laquelle de 80 % à 50 %, notamment de 75 % à 60 %, du poids total des agents émulsifiants, sont du type huile dans eau (H/E).

Dans le latex inverse auto-inversible tel que défini précédemment, la phase huile représente généralement de 15 % à 50 %, de préférence de 20 % à 25 %, de son poids total.

Le latex inverse auto-inversible contient aussi entre 5 % et 60 % en poids, d'eau et plus particulièrement entre 20 % et 50 % en poids d'eau.

Le latex inverse auto-inversible selon l'invention peut également contenir divers additifs tels que des agents complexants ou des agents limiteurs de chaîne.

Par "agent émulsifiant du type eau dans huile", on désigne des agents émulsifiants possédant une valeur HLB suffisamment faible pour fournir des émulsions eau dans huile, tels que les polymères tensioactifs commercialisés sous le nom de HYPERMER^{™} comme l'HYPERMER^{™} B246, l'HYPERMER^{™} B41 ou l'HYPERMER^{™} 2296 ou tels que les esters de sorbitan, comme le monooléate de sorbitan commercialisé par la Société SEPPIC sous le nom MONTANE^{™} 80, l'isostéarate de sorbitan commercialisé par SEPPIC sous le nom MONTANE^{™} 70 ou le sesquioléate de sorbitan commercialisé par SEPPIC sous le nom de MONTANE^{™} 83. Lorsqu'il s'agit d'un mélange d'agents émulsifiants du type eau dans huile, la valeur HLB à prendre en considération est celle dudit mélange.

Par "agent émulsifiant du type huile dans eau", on désigne des agents émulsifiants possédant une valeur HLB suffisamment élevée pour fournir des émulsions huile dans l'eau tels que, par exemple, les esters de sorbitan éthoxylés comme l' oléate de sorbitan éthoxylé à 20 moles d'oxyde d'éthylène, l'huile de ricin éthoxylée à 40 moles d'oxyde d'éthylène, le laurate de sorbitan éthoxylé à 20 moles d'oxyde d'éthylène commercialisés par la société SEPPIC respectivement sous les noms MONTANOX^{™} 80, SIMULSOL^{™} OL 50 et MONTANOX^{™} 20, l'alcool laurique éthoxylé à 7 moles d'oxyde d'éthylène commercialisé par la société SEPPIC sous le nom SIMULSOL^{™} P7, l'alcool oléocétylique décaéthoxylé d'éthylène commercialisé par la société SEPPIC sous le nom SIMULSOL^{™} OC 710 ou les hexaoléates de sorbitan polyéthoxylés commercialisés par la société ATLAS Chemical Industries sous les noms G-1086 et G-1096,les nonylphénol éthoxylés.

La phase huile du latex inverse auto-inversible décrit ci-dessus, est constituée :
soit par une huile minérale commerciale contenant des hydrocarbures saturés de type paraffine, isoparaffine, cycloparaffine, présentant à température ambiante, une densité entre 0,7 et 0,9 et un point d'ébullition supérieur à 180°C, telle que par exemple, l'ISOPAR^{™} M ou l'ISOPAR^{™} L, l'EXXOL^{™} D 100 S commercialisé par EXXON ou les huiles blanches minérales conformes aux réglementations FDA 21 CFR 172.878 et FR 178.3620(a), telles que le MARCOL^{™} 52 ou le MARCOL^{™} 82, également commercialisées par EXXON ;
soit par le polyisobutène hydrogéné, commercialisé en France par la société Ets B. Hossow et Cie sous le nom PARLEAM - POLYSYNLAN^{™} et cité dans Michel and Irene Ash ; Thesaurus of Chemical products, Chemise Publicité Cos, Ince. 1986 Volume I, page 211 (ISBN 0 7131 36030) ;
soit par l'isohexadécane, identifié dans Chemical Abstracts par le numéro RN = 93685 - 80 - 4 et qui est un mélange d'isoparaffines en C₁₂, C₁₆ et C₂₀ contenant au moins 97 % d'isoparaffmes en C₁₆, parmi lesquelles le constituant principal est le 2,2,4,4,6,8,8-heptaméthyl nonane (RN = 4390-04-9), commercialisé en France par la société Bayer ;
soit par l'isododécane, commercialisé en France par la société Bayer;
soit par le squalane qui est identifié dans Chemical Abstracts par le numéro RN = 111-01-3 et qui est un mélange d'hydrocarbures contenant plus de 80 % en poids de 2,6,10,15,19,23-hexaméthyl tétracosane. Il est commercialisé en France par la société SOPHIM, sous le nom PHYTOSQUALANE^{™} ;
soit parmi les esters d'acides gras de formule (II) :

   R₁-(C=O}-O-[[CH₂-CH[O-[C(=O)]ₘ-R₂]-CH₂-O]ₙ-[C(=O)]ₚ]_{q}-R₃ (II)

   dans laquelle R₁ représente une chaîne hydrocarbonée saturée ou insaturée, linéaire ou ramifiée comportant de 7 à 30 atomes de carbone, R₂ représente indépendamment de R₁, un atome d'hydrogène, une chaîne hydrocarbonée saturée ou insaturée linéaire ou ramifiée comportant de 7 à 30 atomes de carbone, R₃ représente indépendamment de R₁ ou de R₂, un atome d'hydrogène ou une chaîne hydrocarbonée saturée ou insaturée, linéaire ou ramifiée comportant de 1 à 30 atomes de carbone, m, n, p et q sont indépendamment l'un de l'autre égaux à 0 ou à 1, étant entendu que lorsque R₃ représente un atome d'hydrogène, q est différent de 0.

Comme composés de formule (II), il y a plus particulièrement les composés de formule (IIa) :

R₁-(C=O)-O-CH₂-CH[O-[C(=O)ₘ-R₂]-CH₂-O-[C(=O)]ₚ-R₃ (IIa)

correspondant à la formule (II) telle que définie précédemment dans laquelle q et n sont égaux à 1, ou un mélange de composés de formules (IIa) ; dans ce cas il s'agit de préférence, ou bien d'un composé de formule (IIa₁) :

R₁-(C=O)-O-CH₂-CH(OH)-CH₂-OH (IIa₁)

correspondant à la formule (IIa) telle que définie précédemment, dans laquelle m et p sont égaux à 0 et R₂ et R₃ représentent un atome d'hydrogène, ou bien un composé de formule (IIa₂) :

R₁-(C=O)-O-CH₂-CH(OH)-CH₂-O-C(=O)-R₃ (IIa₂)

correspondant à la formule (IIa) telle que définie précédemment dans laquelle p est égal à 1, m est égal à 0 et R₂ représente un atome d'hydrogène, ou bien un composé de formule (IIa₃):

R₁-(C=O)-O-CH₂-CH[O-C(=O)-R₂]-CH₂-O-C(=O)-R₃ (IIa3)

correspondant à la formule (IIa) telle que définie précédemment dans laquelle m et p sont égaux à 1, ou bien un mélange de composés de formules (IIa₁), (IIa₂) et/ou (IIa₃).

Comme exemples de composés de formules (IIa₁), (IIa₂) ou (IIa₃), il y a par exemple les triglycérides d'acides gras ou de mélanges d'acides gras tels que le mélange de triglycérides d'acides gras comportant de 6 à 10 atomes de carbone, commercialisé sous le nom SOFTENOL^{™} 3819, le mélange de triglycérides d'acides gras comportant de 8 à 10 atomes de carbone, commercialisé sous le nom SOFTENOL^{™} 3108, le mélange de triglycérides d'acides gras comportant de 8 à 18 atomes de carbone, commercialisé sous le nom SOFTENOL^{™} 3178, le mélange de triglycérides d'acides gras comportant de 12 à 18 atomes de carbone, commercialisé sous le nom SOFTENOL^{™} 3100, le mélange de triglycérides d'acides gras comportant 7 atomes de carbone commercialisé sous le nom SOFTENOL^{™} 3107, le mélange de triglycérides d'acides gras comportant 14 atomes de carbone commercialisé sous le nom SOFTENOL^{™} 3114 ou le mélange de triglycérides d'acides gras comportant 18 atomes de carbone commercialisé sous le nom SOFTENOL^{™} 3118, le dilaurate de glycérol, le dioléate de glycéroI, l'isostéarate de glycérol, le distéarate de glycérol, le monolaurate de glycérol, le monooléate de glycérol, le monoisostéarate de glycérol, le monostéarate de glycérol ou un mélange de ces composés.

Selon un troisième aspect de la présente invention, celle-ci a aussi pour objet un procédé de préparation du latex inverse auto-inversible tel que défini précédemment, caractérisé en ce que:
a) l'on émulsionne une solution aqueuse contenant les monomères et les éventuels additifs, dans une phase huile en présence d'un ou plusieurs agents émulsifiants de type eau dans huile et du monomère tensioactif non ionique,
b) l'on amorce la réaction de polymérisation par introduction dans l'émulsion formée en a), d'un initiateur de radicaux libres et d'éventuellement un co-initiateur puis on la laisse se dérouler,
c) lorsque la réaction de polymérisation est terminée. l'on introduit un ou plusieurs agents émulsifiants de type huile dans eau à une température inférieure à 50°C.

Selon une variante de ce procédé, le milieu réactionnel issu de l'étape b), est concentré par distillation, avant la mise en oeuvre de l'étape c).

Selon une mise en oeuvre préférée du procédé tel que défini précédemment, la réaction de polymérisation est amorcée par un couple oxydoréducteur générateur d'ions kydrogénosulfite (HSO₃) tel que le couple hydroperoxyde de cumène -métabisulfite de sodium (Na₂S₂O₅) ou le couple hydroperoxyde de cumène-chlorure de thionyle (SOCl₂) à une température inférieure ou égale à 10°C, si désiré accompagné d'un agent co-initiateur de polymérisation tel que par exemple l'azo-bis(isobutyronitrile), le dilaurylperoxyde ou le persulfate de sodium puis conduite soit de manière quasi adiabatique jusqu'à une température supérieure ou égale à 50°C, soit en contrôlant la température.

Le polyélectrolyte tel que défini précédemment, peut être isolé du latex inverse auto-inversible précédent, par différents procédés connus de l'homme du métier, comme la technique de précipitation qui consiste à couler le latex dans un large excès de solvant tel que l'acétone, l'isopropanol ou l'éthanol, ou comme la technique de séchage par aspersion, dite de "Spray drying", qui est décrite dans la publication internationale WO 00/01757 ou encore par déshydratation azéotropique.

Selon une variante des procédés de préparation tels que définis précédemment, le polyélectrolyte objet de la présente invention est isolé du latex inverse auto-inversible.

Le polyélectrolyte ou le latex inverse auto-inversible objets de la présente invention, peuvent être mis en oeuvre par exemple comme épaississant de compositions cosmétiques ou pharmaceutiques, comme épaississant de pâtes d'impression pour l'industrie textile, comme épaississants de détergents industriels ou ménagers, comme additifs pour l'industrie pétrolière, comme modificateur de rhéologie pour les boues de l'orage.

Grâce à son caractère cationique le polyélectrolyte objet de la présente invention ainsi que les latex inverses auto-inversibles en comportant, sont avantageusement utilisés comme épaississants et/ou comme émulsionnant dans des compositions cosmétiques ou pharmaceutiques destinées au soin et/ou au conditionnement des cheveux.

De telles compositions se présentent habituellement sous forme de shampoings d'émulsions, de micro-émulsions et notamment dans le cas des conditionneurs d'émulsions vaporisables.

C'est pourquoi, selon un dernier aspect l'invention a pour objet, une composition cosmétique ou pharmaceutique caractérisée en en ce qu'elle contient comme agent émulsionnant et/ou épaississant, une quantité efficace soit du polyélectrolyte cationique tel que défini précédemment, soit du latex inverse auto-inversible en comportant.

Les polyélectrolytes cationiques ou les latex inverses auto-inversibles peuvent être formulés dans des formules cosmétiques, dermo-pharmaceutiques ou pharmaceutiques comme des mousses, des gels, des lotions, des sprays, des shampoings, des conditionneurs, des lotions pour les mains et le corps, des écrans solaires, et plus généralement dans des produits de soin.

Les exemples suivants illustrent la présente invention sans toutefois la limiter.

### A-Exemple de préparation de latex inverses auto-inversible comportant des polvélectrolvtes selon l'invention

### Exemple 1 : Latex inverse auto-inversible du copolvmère AM/APTAC/AL(40E) (84,7/14,9/0,4) réticulé au MBA

On charge dans un bêcher, sous agitation:
- 164,4 g d'eau permutée
- 362,1 g d'une solution commerciale à 50% d'acrylamide (AM)
- 123,9 g d'une solution commerciale à 75% de chlorure d'acrylamido-propyltriméthylammonium (APTAC)
- 0,070 g de méthylène bis acrylamide (MBA)
- 0,45 g d'une solution aqueuse commerciale à 40% du sel de sodium de l'acide diéthylènetriamine pentaacétique.

On prépare une phase huile en mélangeant successivement :
- 259 g d'isohexadécane
- 20 g d'isostéarate de sorbitan (MONTANE^{™} 70)
- 5 g d'HYPERMER^{™} 2296 (Uniquema)
- 5 g d'acrylate de lauryle tétraéthoxylé [AL(40E)]
- 0.1 g d'azo-bis(isobutyronitrile) (AIBN).

La phase aqueuse est incorporée progressivement dans la phase organique puis soumise à une agitation mécanique violente au moyen d'une turbine de type ULTRA-TURRAX^{™}, pour former une émulsion inverse (eau/huile).

L'émulsion est ensuite refroidie jusqu'à environ 10°C et placée sous barbotage d'azote pendant environ 60 minutes pour en éliminer l'oxygène. La polymérisation est alors initiée en y incorporant 10 cm³ d'une solution d'hydroperoxyde de cumène à 0,68 % en poids dans l'isohexadecane. Après homogénéisation du milieu, on ajoute 25 g d'une solution aqueuse de métabisulfite de sodium à 0,1 % en poids en laissant monter la température du mélange jusqu'à la température finale de polymérisation puis en laissant le mélange pendant 90 minutes. L'ensemble est ensuite refroidi jusqu'à 35° C environ, puis on ajoute 40 g d'alcool laurique éthoxylé à 7 moles (SIMULSOL^{™} P7). On obtient le latex inverse auto inversible désiré.

### Analyse

Teneur en polyélectrolyte: environ 27,5 % poids

### Mesure de viscosité

Viscosité d'une solution aqueuse à 3 % en poids du latex inverse auto-inversible (Brookfield RVT, Mobile 6: ; Vitesse: 5 tours par minute) : η = 45 000 mPa.s

Viscosité d'une solution aqueuse à 3% en poids du latex inverse auto-inversible et à 1 ‰ de chlorure de sodium

(Brookfield RVT, Mobile 3; Vitesse: 5 tours par minute) : η = 1 280 mPa.s

### Exemples de formulations.

### Exemple 2 : Soin an ti-stress pour cheveux

### Formule

| | |
|---|---|
| Phase A | |
| Eau | QSP 100 % |
| Gomme de xanthane | 0.50 % |
| Phase B | |
| SEPICAP^{™} MP | 3.00 % |
| Phase C | |
| Composition de l'Exemple 1 | 4.00 % |
| | |
| Phase D | |
| Butylène Glycol | 5.00 % |
| LANOL^{™} 99 | 5.00 % |
| SEPICIDE^{™} HB | 0.30 % |
| SEPICIDE^{™} CI | 0.20 % |
| Parfum | 0.20 % |

### Mode Opératoire

Disperser la gomme xanthane dans l'eau avec une défloculeuse. Ajouter ensuite SEPICAP^{™} MP, puis la composition de l'exemple 1 ; la disperser puis ajouter les ingrédients de la phase D.

### Exemple 3 : Savon liquide antiseptique

### Formule

| | | |
|---|---|---|
| Phase A | MONTALINE^{™} C 40 | 30.00 % |
| | ORAMIX^{M} NS 10 | 15.00 % |
| | Chlorhexidine digluconate (à 20 %) | 5.00 % |
| | Composition de l'exemple 1 | 2.00 % |
| Phase B | Eau | QSP 100 % |

Les définitions des produits commerciaux utilisés dans les exemples sont les suivantes: SEPICIDE^{™} HB est un mélange conservateur comprenant du Phenoxyethanol, du méthylparaben, de l'éthylparaben, du propylparaben et du butyl paraben, commercialisé par la société SEPPIC.

SEPICIDE^{™} CI est de l'imidazolidinyl urée, commercialisé par la société SEPPIC. ORAMIX^{™} NS 10 : Decyl glucoside commericalisé par SEPPIC.

MONTALINE^{™} C40 : (cocamoniumcarbamoyl chloride) commercialisé par SEPPIC. SEPICAP^{™} MP : (Sodium cocoyl amino acids 1 potassium dimethicone copolyol panthényl phosphate) commercialisé par SEPPIC.

LANOL^{™} 99: (isononyl isononanoate) commercialisé par SEPPIC

## Revendications

1. Polyélectrolyte cationique linéaire ou réticulé, **caractérisé en ce qu'**il est obtenu par copolymérisation d'au moins un monomère cationique avec au moins un monomère neutre et au moins un monomère tensioactif non ionique, choisi parmi les composés représentés par la formule générale (I) :
A-C(==O)-O-[(CH₂-CH(R₁)-O]ₙ-R (I)
dans laquelle :
- A représente le radical vinyle ou le radical 2-propènyle ;
- R représente un radical aliphatique hydrocarboné, linéaire ou ramifié, saturé ou insaturé comprenant de 8 à 18 atomes de carbone ;
- R₁ représente un atome d'hydrogène ;
- n représente un nombre compris entre 1 et 10.
et dans lequel :
- de 5 % à 35 % des motifs monomériques qu'il comprend, est un monomère cationique,
- de 55 % à 95 % des motifs monomériques qu'il comprend, est un monomère neutre, et
- de 0,1 % à 5 % des motifs monomériques qu'il comprend, est un monomère tensioactif non ionique de formule (I).

2. Polyélectrolyte tel que défini à la revendication 1, dans lequel les monomères neutres sont choisis parmi l'acrylamide, le méthacrylamide, le vinylpyrrolidone, le diacétone-acrylamide, le diméthylacrylamide, l'acrylate de (2-hydroxy éthyle), l'acrylate de (2,3-dihydroxy propyle), le méthacrylate de (2-hydroxy éthyle), le méthacrylate de (2,3-dihydroxy propyle) ou un dérivé éthoxylé de poids moléculaire compris entre 400 et 1 000, de chacun de ces esters.

3. Polyélectrolyte tel que défini à l'une quelconque des revendications 1 ou 2, dans lequel les monomères cationiques sont choisis parmi le chlorure de 2,N,N,Ntétraméthyl 2-[(1-oxo 2-propènyl) amino] propanammonium (AMPTAC), le chlorure de 2,N,N-triméthyl 2-[(1-oxo 2-propènyl) amino] propanammonium, le chlorure de N,N,Ntriméthyl 3-[(1-oxo 2-propènyl) amino] propanammonium (APTAC), le chlorure de diallyl diméthylammonium (DADMAC), le chlorure de N,N,N-triméthyl 2-[(I-oxo 2-propènyl)] éthanammonium, le chlorure de N,N,N-triméthyl 2-[(1-oxo 2- méthyl 2-propènyl)] éthanammonium, le N-[2-(diméthylamino) 1,1-diméthyl] acrylamide, le N-[2(méthylamino) 1, 1-diméthyl] acrylamide, l'acrylate de 2-(diméthylamino) éthyle, le méthacrylate de 2-(diméthylamino) éthyle ou le N-[3-(diméthylamino) propyl] acrylamide.

4. Composition comprenant une phase huile, une phase aqueuse, au moins un agent émulsifiant de type eau dans huile (E/H), au moins un agent émulsifiant de type huile dans eau (H/E), sous forme d'un latex inverse auto-inversible comprenant de 20 % à 70 % en poids, et de préférence de 25 % à 40 % en poids, d'un polyélectrolyte cationique tel que défini à l'une quelconque des revendications 1 à 3.

5. Utilisation du polyélectrolyte ou du latex inverse auto-inversible dudit polyélectrolyte tels que définis à l'une quelconque des revendications 1 à 4, comme épaississant de compositions cosmétiques ou pharmaceutiques, comme épaississant de pâtes d'impression pour l'industrie textile, comme épaississants de détergents industriels ou ménagers, comme additifs pour l'industrie pétrolière ou comme modificateur de rhéologie pour les boues de forage.

6. Utilisation selon la revendication 5, comme épaississants et/ou comme émulsionnant dans des compositions cosmétiques ou pharmaceutiques destinées au soin et/ou au conditionnement des cheveux.

7. Composition cosmétique ou pharmaceutique **caractérisée en** en ce qu'elle contient comme agent émulsionnant et/ou épaississant, une quantité efficace, soit du polyélectrolyte cationique, soit du latex inverse auto-inversible tels que définis à l'une des revendications 1 à 4.

## Claims

1. Linear or crosslinked cationic polyelectrolyte, **characterized in that** it is obtained by copolymerization of at least one cationic monomer with at least one neutral monomer and at least one nonionic surfactant monomer which is chosen from the compounds represented by general formula (I):
**A-C(=O)-O-[(CH₂-CH(R₁)-O]ₙ-R** (I)
in which:
- A represents the vinyl radical or the 2-propenyl radical;
- R represents a saturated or unsaturated, linear or branched, aliphatic hydrocarbon radical comprising from 8 to 18 carbon atoms;
- R₁ represent a hydrogen atom;
- n represents a number between 1 and 10 and in which:
- from 5% to 35% of the monomeric units which it comprises is a cationic monomer,
- from 55% to 95% of the monomeric units which it comprises is a neutral monomer, and
- from 0.1% to 5% of the monomeric units which it comprises is a nonionic surfactant monomer of formula (I).

2. Polyelectrolyte as defined in Claim 1, in which the neutral monomers are chosen from acrylamide, methacrylamide, vinylpyrrolidone, diacetone-acrylamide, dimethylacrylamide, (2-hydroxyethyl) acrylate, (2,3-dihydroxypropyl) acrylate, (2-hydroxyethyl) methacrylate, (2,3-dihydroxypropyl) methacrylate or an ethoxylated derivative having a molecular weight of between 400 and 1000, of each of these esters.

3. Polyelectrolyte as defined in either of Claims 1 and 2, in which the cationic monomers are chosen from 2,N,N,N-tetramethyl-2-[(1-oxo-2-propenyl)-amino]propanammonium chloride (AMPTAC), 2,N,N-trimethyl-2-[(1-oxo-2-propenyl)amino]propanammonium chloride, N,N,N-trimethyl-3-[(1-oxo-2-propenyl)amino]propanammonium chloride (APTAC), diallyldimethylammonium chloride (DADMAC), N,N,N-trimethyl-2-[(1-oxo-2-propenyl)]ethan-ammonium chloride, N,N,N-trimethyl-2-[(1-oxo-2-methyl-2-propenyl)]ethanammonium chloride, N-[2-(dimethyl-amino)-1,1-dimethyl] acrylamide, N-[2-(methylamino)-1,1-dimethyl]acrylamide, 2-(dimethylamino)ethyl acrylate, 2-(dimethylamino)ethyl methacrylate or N-[3-(dimethyl-amino)propyl]acrylamide.

4. Composition comprising an oily phase, an aqueous phase, at least one water-in-oil (W/O) type emulsifying agent, at least one oil-in-water (O/W) type emulsifying agent, in the form of a self-reversible invert latex comprising from 20% to 70% by weight, preferably from 25% to 40% by weight, of a cationic polyelectrolyte as defined in any one of Claims 1 to 3.

5. Use of the polyelectrolyte or of the self-reversible invert latex of the said polyelectrolyte as defined in any one of Claims 1 to 4, as a thickener for cosmetic or pharmaceutical compositions, as a thickener for printing pastes for the textile industry, as thickeners for industrial or household detergents, as additives for the petroleum industry, or as a rheology modifier for drilling mud.

6. Use according to Claim 5, as thickeners and/or as emulsifiers in cosmetic or pharmaceutical compositions intended for hair care and/or conditioning.

7. Cosmetic or pharmaceutical composition, **characterized in that** it contains, as emulsifying and/or thickening agent, an effective quantity either of the cationic polyelectrolyte or of the self-reversible invert latex as defined in one of Claims 1 to 4.

## Patentansprüche

1. Linearer oder vernetzter kationischer Polyelektrolyt, **dadurch gekennzeichnet, daß** er durch Copolymerisation von mindestens einem kationischen Monomer mit mindestens einem neutralen Monomer und mindestens einem nichtionischen Tensidmonomer, ausgewählt unter den Verbindungen der allgemeinen Formel (I):
A-C (=O) -O- [(CH₂-CH (R₁) -O]ₙ-R (I)
worin:
- A für den Vinylrest oder den 2-Propenylrest steht;
- R für einen gesättigten oder ungesättigten, linearen oder verzweigten, aliphatischen Kohlenwasserstoffrest mit 8 bis 18 Kohlenstoffatomen steht;
- R₁ für ein Wasserstoffatom steht;
- n für eine Zahl zwischen 1 und 10 steht; erhalten wird;
und worin:
- 5 bis 35% der Monomereinheiten, die er enthält, ein kationisches Monomer sind,
- 55 bis 95% der Monomereinheiten, die er enthält, ein neutrales Monomer sind und
- 0,1 bis 5% der Monomereinheiten, die er enthält, ein nichtionisches Tensidmonomer der Formel (I) sind.

2. Polyelektrolyt nach Anspruch 1, worin die neutralen Monomere unter Acrylamid, Methacrylamid, Vinylpyrrolidon, Diacetonacrylamid, Dimethylacrylamid, (2-Hydroxyethyl)acrylat, (2,3-Dihydroxypropyl)acrylat, (2-Hydroxyethyl)methacrylat, (2,3-Dihydroxypropyl)methacrylat oder einem ethoxylierten Derivat jedes dieser Ester mit einem Molekulargewicht zwischen 400 und 1000 ausgewählt sind.

3. Polyelektrolyt nach einem der Ansprüche 1 oder 2, worin die kationischen Monomere unter 2,N,N,N-Tetramethyl-2-[(1-oxo-2-propenyl)amino]propan-ammoniumchlorid (AMPTAC), 2,N,N-Trimethyl-2-[(1-oxo-2-propenyl)amino]propanammoniumchlorid, N,N,N-Trimethyl-3-[(1-oxo-2-propenyl)amino]propanammonium-chlorid (APTAC), Diallyldimethylammoniumchlorid (DADMAC), N,N,N-Trimethyl-2-[(1-oxo-2-propenyl)]-ethanammoniumchlorid, N,N,N-Trimethyl-2-[(1-oxo-2-methyl-2-propenyl)]ethanammoniumchlorid, N-[2-(Dimethylamino)-1,1-dimethyl]acrylamid, N-[2-(Methylamino) -1,1-dimethyl]acrylamid, 2-(Dimethylamino)ethylacrylat, 2-(Dimethylamino)ethylmethacrylat oder N-[3-(Dimethylamino)propyl]acrylamid ausgewählt sind.

4. Zusammensetzung, enthaltend eine Ölphase, eine wäßrige Phase, mindestens einen Emulgator vom Wasser-in-Öl-Typ (W/O-Typ), mindestens einen Emulgator vom Öl-in-Wasser-Typ (O/W-Typ), in Form eines selbstinvertierenden inversen Latex, der 20 bis 70 Gew.-% und vorzugsweise 25 bis 40 Gew.-% eines kationischen Polyelektrolyts nach einem der Ansprüche 1 bis 3 enthält.

5. Verwendung des Polyelektrolyts oder des selbstinvertierenden inversen Latex des Polyelektrolyts nach einem der Ansprüche 1 bis 4 als Verdickungsmittel für kosmetische oder pharmazeutische Zusammensetzungen, als Verdickungsmittel für Druckpasten für die Textilindustrie, als Verdickungsmittel für Industrie- oder Haushaltsreinigungsmittel, als Additive für die Erdölindustrie oder als Rheologiemodifikator für Bohrschlämme.

6. Verwendung nach Anspruch 5 als Verdickungsmittel und/oder Emulgator in kosmetischen oder pharmazeutischen Zusammensetzungen für die Haarpflege und/oder -konditionierung.

7. Kosmetische oder pharmazeutische Zusammensetzung, **dadurch gekennzeichnet, daß** sie als Emulgator und/oder Verdickungsmittel eine wirksame Menge des kationischen Polyelektrolyts oder des selbstinvertierenden inversen Latex nach einem der Ansprüche 1 bis 4 enthält.
